# EUROPEAN PATENT SPECIFICATION

(11) **EP 1 511 563 B1**
(45) Date of publication and mention of the grant of the patent: **28.11.2012**
(21) Application number: 03713718.9
(22) Date of filing: 25.02.2003
(51) Int. Cl.: B01J 23/66, B01J 35/10, B01J 37/00, C07D 301/10, C07C 23/10, C07C 213/04

(54) **SUPPORTED SILVER CATALYST AND AN EPOXIDATION PROCESS USING THE CATALYST**
SILBERKATALYSATOR AUF EINEM TRÄGER UND SEINE VERWENDUNG IN EINEM EPOXIDIERUNGSVERFAHREN
CATALYSEUR A BASE D'ARGENT SUR SUPPORT ET SON UTILISATION DANS UN PROCEDE D'EPOXYDATION

(30) Priority: 25.02.2002 US 360060 P
(43) Date of publication of application: 09.03.2005
(73) Proprietor: Shell Internationale Research Maatschappij B.V., 2596 HR Den Haag (NL)
(72) Inventor: LOCKEMEYER, John, Robert, Sugar Land, TX 77479 (US); YEATES, Randall, Clayton, Sugar Land, TX 77478 (US); SZYMANSKI, Thomas, Hudson, OH 44236 (US); REMUS, Donald, James, Stow, OH 44224 (US); GERDES, William, Herman, Hudson, OH 44236 (US)
(86) International application number: PCT/US2003/005902
(87) International publication number: WO 2003/072246

(56) References cited:
- EP-A- 0 480 538
- EP-A- 0 496 470
- WO-A-97/40932
- US-A- 3 664 970
- US-A- 4 761 394
- US-A- 4 845 296
- US-A- 4 908 343
- US-A- 5 063 195

## Description

### BACKGROUND OF THE INVENTION

The present invention relates to a catalyst and to the use of the catalyst in olefin epoxidation.

In olefin epoxidations, catalyst performance may be assessed on the basis of selectivity, activity and stability of operation. The selectivity is the percentage of the converted olefin yielding the desired olefin oxide. As the catalyst ages, the percentage of the olefin converted normally decreases with time and to maintain a constant level of olefin oxide production the temperature of the reaction is increased. However this adversely affects the selectivity of the conversion to the desired olefin oxide. In addition, the equipment used can tolerate temperatures only up to a certain level so that it is necessary to terminate the reaction when the reaction temperature would reach a level inappropriate for the reactor. Thus the longer the selectivity can be maintained at a high level and the epoxidation can be performed at an acceptably low temperature, the longer the catalyst charge can be kept in the reactor and the more product is obtained. Quite modest improvements in the maintenance of selectivity over long periods yields huge dividends in terms of process efficiency.

Olefin epoxidation catalysts comprise a silver component, usually with one or more further elements codeposited therewith on a carrier. Carriers are typically formed of a temperature resistant oxide such as α-alumina and in general higher purity has been found to correlate with better performance. However it has also been found for example that the presence of minor amounts of impurities in the carrier such as alkali metals and some forms of silica can have a beneficial effect.

Intuitively it might also be considered that the higher the surface area of the carrier, the greater the area available for deposition of the silver and therefore the more effective the silver deposited thereon. However, this is generally found not to be the case and in modern catalysts the tendency is to use a carrier with a surface area of less than 1 m²/g.

US-A-4908343 discloses silver catalysts comprising promoters such as cesium and, for example, other alkali metals. Carrier E disclosed therein contains at least 99.0 weight percent alpha alumina and about 0.2 weight percent fluoride. Said carrier has a surface area of 1.17 m²/g and the pore size distribution is disclosed in US-A-4908343.
EP-A-0480538 describes silver-containing, supported catalysts for the epoxidation of alkene to the corresponding alkylene oxide, which contain a stability and/or activity enhancing amount of a cobalt-containing compound.
US-A-4761394 discloses an ethylene oxide catalyst having an improved selectivity which catalyst comprises silver, a promoting amount of alkali metal and a promoting amount of rhenium supported on a porous refractory support. Carrier F in Table 1 comprises 70-75 %wt. alpha alumina and 25-30 wt. % silica. Said carrier has a surface area of 2.06 m²/g and the pore size distribution curve is given in Figure 7.
WO-A-97/40932 relates to the preparation of carriers for use in catalysts. Said document discloses a process for the production of an alpha alumina-based catalyst carrier, which does not require the presence of pore-inducing burnout materials.

### SUMMARY OF THE INVENTION

The present invention teaches that the picture with respect to carrier surface area is significantly more complicated than was at first appreciated since the nature of the porosity of the carrier, in particular the pore size distribution and the pore volume provided by the pores which have a pore size within a defined range, has now been found to play a significant role. On this basis it was possible to prepare olefin epoxidation catalysts with excellent activity, selectivity and unusually prolonged retention of the activity and stability level. The carriers having an advantageous pore size distribution may be made from particulate materials which have specific particle sizes.

The present invention provides a catalyst which comprises a carrier and silver deposited on the carrier in a quantity of at least 10 g/kg, which carrier has a surface area in the range of from 1.6 m²/g to at most 2.9 m²/g, and a pore size distribution such that pores with diameters in the range of from 0.2 to 10 µm represent more than 80 % of the total pore volume and such pores together provide a pore volume of at least 0.27 ml/g, relative to the weight of the carrier, wherein the carrier comprises at least 95 %w α-alumina.

The invention also provides a process for the preparation of a catalyst which process comprises:
a) selecting a carrier which has a surface area of in the range of from 1.6 m²/g to at most 2.9 m²/g, and a pore size distribution such that pores with diameters in the range of from 0.2 to 10 µm represent more than 80 % of the total pore volume and such pores together provide a pore volume of at least 0.27 ml/g, relative to the weight of the carrier wherein the carrier comprises at least 95 % wt α-alumina, and
b) depositing the silver on the carrier.

Preferably the carrier has been obtained by a method which comprises forming a mixture comprising:
a) from 50 to 90 %w of a first particulate α-alumina having an average particle size (d₅₀) of from more than 10 up to 100 µm; and
b) from 10 to 50 %w of a second particulate α-alumina having a d₅₀ of from 1 to 10 µm;
%w being based on the total weight of α-alumina in the mixture; and firing the mixture to form the carrier. In an embodiment, amongst others, the mixture may be shaped into formed bodies and the formed bodies are fired to form the carrier. When the formed bodies are formed by extrusion, it may be desirable to include conventional burnout materials and/or extrusion aids, and an aqueous liquid, e.g. water, in the mixture.

Further, the invention provides a process for the epoxidation of an olefin, which process comprises reacting an olefin with oxygen in the presence of a catalyst which comprises a carrier and silver deposited on the carrier in a quantity of at least 10 g/kg which carrier has a surface area in the range of from 1.6 m²/g to at most 2.9 m²/g, and a pore size distribution such that pores with diameters in the range of from 0.2 to 10 µm represent more than 80 % of the total pore volume and such pores together provide a pore volume of at least 0.27 ml/g, relative to the weight of the carrier wherein the carrier comprises at least 95% w α-alumina.

The invention also provides a method of using an olefin oxide for making a 1,2-diol, a 1,2-diol ether or an alkanolamine comprising converting the olefin oxide into the 1,2-diol, the 1,2-diol ether or the alkanolamine wherein the olefin oxide has been obtained by a process for the epoxidation of an olefin in accordance with this invention.

In accordance with the teaching of this invention, by maximizing the number of pores having a diameter in the range of 0.2 to 10 µm, in particular by minimizing the number of pores having a diameter greater than 10 µm, the catalyst is advantaged over catalysts where are prepared from carriers which have a substantial number of pores having a diameter greater than 10 µm.

### DETAILED DESCRIPTION OF THE INVENTION

"Surface area" as used herein is understood to refer to the surface area as determined by the BET (Brunauer, Emmett and Teller) method as described in Journal of the American Chemical Society 60 (1938) pp. 309-316.

As used herein, water absorption is deemed to have been measured in accordance with ASTM C393, and water absorption is expressed as the weight of the water that can be absorbed into the pores of the carrier, relative to the weight of the carrier.

The pore size distribution may be measured by a conventional mercury intrusion device in which liquid mercury is forced into the pores of a carrier. Greater pressure is needed to force the mercury into the smaller pores and the measurement of pressure increments corresponds to volume increments in the pores penetrated and hence to the size of the pores in the incremental volume. As used herein, the pore size distribution and the pore volumes are as measured by mercury intrusion to a pressure of 3.0 x 10⁸ Pa using a Micromeretics Autopore 9200 model (130° contact angle, mercury with a surface tension of 0.473 N/m, and correction for mercury compression applied).

The average particle size, referred to herein as "d₅₀", is as measured by a Horiba LA900 particle size analyzer and represents a particle diameter at which there are equal spherical equivalent volumes of particles larger and particles smaller than the stated average particle size. The method includes dispersing the particles by ultrasonic treatment, thus breaking up secondary particles into primary particles. This sonification treatment is continued until no further change in the d₅₀ value is noticed, which typically requires 5 minute sonification when using the Horiba LA900 particle size analyzer.

As used herein, pore volume (ml/g), surface area (m²/g) and water absorption (g/g) are defined relative to the weight of the carrier, unless stated otherwise.

Typically, the pore size distribution is such that pores with diameters less than 0.2 µm represent less than 10 % of the total pore volume. Typically, the pore size distribution is such that pores with diameters greater than 10 µm represent less than 20 %, in particular less than 15 %, of the total pore volume. The pore size distribution is such that the pores with diameters in the range of from 0.2 to 10 µm represent more than 80 %, more preferably more than 85 %, most preferably more than 90 % of the total pore volume; the pores with diameters greater than 10 µm represent less than 12 %, in particular less than 10 %, more in particular less than 5 % of the total pore volume; and the pores with diameters less than 0.2 µm represent less than 7 %, in particular at most 5 %, and more in particular at most 4 % of the total pore volume.

Frequently, the pore size distribution is such that the pores with diameters in the range of from 0.2 to 10 µm represent less than 99.9 %, more frequently less than 99 % of the total pore volume; the pores with diameters greater than 10 µm represent more than 0.1 %, more frequently more than 0.5 % of the total pore volume; and the pores with diameters less than 0.2 µm represent more than 0.1 %, more frequently more than 0.5 % of the total pore volume.

Typically, the pores with diameters in the range of from 0.2 to 10 µm provide a pore volume of at least 0.28 ml/g, in particular at least 0.3 ml/g, more in particular at least 0.35 ml/g. Typically, the pores with diameters in the range of from 0.2 to 10 µm provide a pore volume of at most 0.8 ml/g, more typically at most 0.7 ml/g, in particular at most 0.6 ml/g, more in particular at most 0.56 ml/g. In a preferred embodiment, the pores with diameters in the range of from 0.2 to 10 µm provide a pore volume in the range of from 0.35 to 0.45 ml/g.

The total pore volume of the carrier may vary between wide ranges. Typically the total pore volume is at least 0.28 ml/g, in particular at least 0.30 ml/g, more in particular at least 0.35 ml/g. Typically, the total pore volume is at most 0.8 ml/g, and more typically it is at most 0.7 ml/g, in particular at most 0.6 ml/g, more in particular at most 0.56 ml/g. In a preferred embodiment, the total pore volume of the carrier is in the range of from 0.35 to 0.45 ml/g.

The surface area of the carrier is at least 1.6 m²/g. The surface area is at most 2.9 m²/g. Preferably, the surface area is in the range of from 1.6 to 2.6 m²/g, most preferably from 1.6 to 2.2 m²/g, for example from 1.6 to 2.0 m²/g or from 1.8 to 2.2 m²/g.

The water absorption of the carrier is typically at least 0.3 g/g, more typically at least 0.35 g/g. Frequently, the water absorption is at most 0.8 g/g, more frequently at most 0.7 g/g, or at most 0.55 g/g. Preferably, the water absorption of the carrier is in the range of from 0.35 to 0.7 g/g, for example 0.39 g/g, or 0.41 g/g, or any value in the range of from 0.35 to 0.55 g/g, in particular 0.38 to 0.5 g/g or 0.40 to 0.55 g/g or 0.48 to 0.55 g/g. A higher water absorption and a higher total pore volume are in favour in view of a more efficient deposition of silver and further elements, if any, on the carrier by impregnation. However, at a higher water absorption and higher total pore volume, the carrier, or the catalyst made therefrom, may have lower crush strength.

The carrier comprises at least 95 %w α-alumina, frequently up to 99.9 %w α-alumina.

Carriers may generally be made by firing particulate mineral components at an elevated temperature until the particles sinter together. In general, firing is continued until the particles are bonded together, either by the formation of bond posts from any added bond material or through sintering, but preferably not beyond the point at which the water absorption of the carrier is reduced.

The water absorption of the carrier may suitably be controlled by the use of so-called burnout materials, and it is preferred to employ a burnout material when practicing this invention. Burnout materials are well known in the art (cf., for example, F F Y Wang (Ed.), "Treatise on Materials Science and Technology", Volume 9, (New York, 1976), pp. 79-81; or J S Reed, "Introduction to the Principles of Ceramic Processing", (New York, 1988), pp. 152 ff.). The burnout materials may also be used to ensure the preservation of the structure during a green, i.e. unfired, phase of the carrier preparation, for example the phase in which formed bodies are shaped, for example by extrusion. The burnout materials are removed during the firing. The use of burnout materials also allows more complete sintering without too great a reduction in water absorption of the carrier. The burnout materials are typically finely divided solid organic materials that volatilize or burn, leaving as little residue as possible. The selection of the burnout material is considered not to be of any criticality to the invention. Burnout materials may be selected from the group of carbohydrates, gums, flours, proteins, lignins, resins, waxes, alcohols, and esters.

It is also a common expedient to use a bond material, i.e. a material which reduces the length of sintering time applied to bond the particles together, and it is preferred to employ such bond material when practicing this invention. It is advantageous that the bond material also forms a coating on at least a part of the carrier surface, which makes the carrier surface more receptive. Suitable bond materials have been specified hereinafter.

It has been found that, suitably, alumina carriers for use in this invention may be made by a method which comprises forming a mixture comprising:
a) from 50 to 90 %w of a first particulate α-alumina having a d₅₀ of from more than 10 up to 100 µm, in particular from 11 to 60 µm, more in particular from 12 to 40 µm;
b) from 10 to 50 %w of a second particulate α-alumina having a d₅₀ of from 1 to 10 µm, in particular from 2 to 6 µm; and preferably in addition.
c) a bond material;
%w being based on the total weight of α-alumina in the mixture; and then shaping the mixture into formed bodies and firing the formed bodies, typically at a temperature of from 1250 to 1500 °C, to form the carrier.

The present method for making alumina carriers is well adapted to produce the carriers for use in this invention, in view of the careful matching of large and small particles of the α-alumina components. The alumina particles are readily commercially available, or they may readily be made, for example, by subjecting more course materials to grinding and sieving operations. In an embodiment, the smaller particles may be prepared from the larger particles by grinding, and the ground and un-ground particles are then combined. In another embodiment, the desired mixture of large and small particles may be formed by grinding relatively large particles to the extent that the mixture of particles has the desired bimodal particle size distribution.

Typically, the first particulate α-alumina is employed in a quantity of from 65 to 75 %w, relative to the total weight of α-alumina in the mixture. Typically, the second particulate α-alumina is employed in a quantity of from 25 to 35 %w, relative to the total weight of α-alumina in the mixture.

It is preferred that the bond material is based on a silica-containing composition comprising a crystallization inhibitor, inhibiting the formation of crystalline silica-containing compositions. It is also preferred that the bond material provides a coating of a non-crystalline silica compound to the carrier surface.

Typically, silica-containing compositions for use as a bond material comprise an amorphous silica compound which may be, for example, a silica sol, a precipitated silica, an amorphous silica, or an amorphous alkali metal silicate or aluminasilicate. Typically, silica-containing compositions for use as a bond material may also comprise hydrated alumina. The crystallization inhibitor that is most conveniently incorporated is an alkali metal compound, in particular a water soluble salt, such as a sodium or potassium salt.

A convenient bond material may comprise a mixture of boehmite, ammonium silicate or silica sol, and a water soluble sodium salt. Similar effects can be achieved by incorporation of conventional ceramic bonds formulated to contain aluminosilicates and an alkali metal component.

It is preferred that the bond material is based on
a) from 1 to 10 %w, in particular 2 to 5 %w, of an alumina hydrate, calculated as aluminum oxide relative to the weight of the α-alumina;
b) from 0.1 to 1 %w, in particular 0.2 to 0.8 %w, of an amorphous silica compound, as specified hereinbefore, calculated as silicon oxide relative to the weight of the α-alumina; and
c) from 0.01 to 0.5 %w, in particular 0.05 to 0.3 %w, of an alkali metal compound, calculated as the alkali metal oxide relative to the weight of the α-alumina.

In a preferred embodiment, the alumina carrier
may be made by a method which comprises forming a mixture comprising:
a) from 65 to 75 %w, relative to the total weight of α-alumina in the mixture, of a first particulate α-alumina having a d₅₀ of from 10 to 60 µm, in particular from 12 to 40 µm;
b) from 25 to 35 %w, relative to the total weight of α-alumina in the mixture, of a second particulate α-alumina having a d₅₀ of from 2 to 6 µm;
c) from 2 to 5 %w of an alumina hydrate, calculated as aluminum oxide relative to the total weight of α-alumina in the mixture;
d) from 0.2 to 0.8 %w of an amorphous silica compound, as specified hereinbefore, calculated as silicon oxide relative to the total weight of α-alumina in the mixture; and
e) from 0.05 to 0.3 %w, of an alkali metal compound, calculated as the alkali metal oxide relative to the total weight of α-alumina in the mixture;
and then shaping the mixture into formed bodies and firing the formed bodies at a temperature of from 1250 to 1500 °C to form the carrier.

The preferred alumina hydrate is boehmite, though gibbsite, bayerite or diaspore may also be used.

Suitable alkali metals are, for example, lithium, sodium and potassium, or combination thereof. Suitable alkali metal compounds are, for example, alkali metal carbonates, alkali metal acetates, alkali metal formates, alkali metal nitrates, and combinations thereof. Typically, the overall atomic ratio of silicon to the alkali metal is in the range of from 1 to 10, more typically 2 to 8, for example 6. The overall atomic ratio of silicon to the alkali metal is deemed to relate to the total alkali metal content and the total silicon content of the carrier, which includes any alkali metal and any silicon which may be present in the carrier other than in the bond material.

It is also preferred that the carrier particles be prepared in the form of formed bodies, the size of which is in general determined by the dimensions of a reactor in which they are to be deposited. Generally however it is found very convenient to use particles such as formed bodies in the form of powdery particles, trapezoidal bodies, cylinders, saddles, spheres, doughnuts, and the like. The cylinders may be solid or hollow, straight or bend, and they may have their length and cross-sectional dimensions about the same and from 5 to 10 mm.

The formed bodies can be formed from the mixture by any convenient molding process, such as spraying, spray drying, agglomeration or pressing, but preferably they are formed by extrusion of the mixture. For applicable methods, reference may be made to, for example, US-A-5145824, US-A-5512530, US-A-5384302, US-A-5100859 and US-A-5733842. To facilitate such molding processes, in particular extrusion, the mixture may suitably be compounded with up to about 30 %w and preferably from 2 to 25 %w, based on the weight of the mixture, of extrusion aids. Extrusion aids (also referred to by the term "processing aids") are known in the art (cf., for example, "Kirk-Othmer Encyclopedia of Chemical Technology", 4th edition, Volume 5, pp. 610 ff.). Suitable extrusion aids may be for example petroleum jelly, hydrogenated oil, synthetic alcohol, synthetic ester, glycol, polyolefin oxide or polyethylene glycol. Burnout materials are typically applied in a quantity of up to 30 %w, in particular from 2 to 25 %w, relatively to the weight of the mixture. Boric acid may also be added to the mixture, for example in a quantity of up to 0.5 %w, more typically in a quantity of from 0.01 to 0.5 %w. The effect of the presence of boric acid may be a reduced content of leachable alkali metal ions in the carrier after firing. Enough water may be added to the mixture to make the mixture extrudable (by the term "the weight of the mixture", as used hereinbefore, is meant the weight of the total mixture, but excluding the weight of any added water).

The formed bodies are dried and fired at a temperature high enough to ensure that the alumina particles are joined together by a sintering action and/or by the formation of bond posts formed from the bond material, if incorporated in the mixture. Generally, drying may take place between 20 and 400 °C and preferably between 30 and 300 °C, typically for a period of up to 100 hours and preferably for from 5 minutes to 50 hours. Typically, drying is performed to the extent that the mixture contains less than 2 %w of water. Generally, firing may take place between 1250 and 1500 °C, typically between 1250 and 1470 °C, preferably between 1300 and 1450 °C, more preferably between 1300 and 1440 °C, typically for a period of up to about 5 hours and preferably for from 2 to 4 hours. Drying and firing may be carried out in any atmosphere, such as in air, nitrogen, or helium, or mixtures thereof. Preferably, in particular when the formed bodies contain organic material, the firing is at least in part or entirely carried out in an oxidizing atmosphere, such as in oxygen containing atmosphere.

It has been found that the performance of the catalyst may be enhanced if the carrier is washed, to remove soluble residues, before deposition of other catalyst ingredients on the carrier. On the other hand, unwashed carriers may also be used successfully. A useful method for washing the carrier comprises washing the carrier in a continuous fashion with hot, demineralised water, until the electrical conductivity of the effluent water does not further decrease. A suitable temperature of the demineralised water is in the range of 80 to 100 °C, for example 90 °C or 95 °C. Reference may be made to WO-00/15333 and US-B-6368998.

Generally, the catalyst of this invention comprises silver as a catalytically active metal. Appreciable catalytic activity is obtained by employing a silver content of the catalyst of at least 10 g/kg, in particular at least 50 g/kg, relative to the weight of the catalyst. The preparation of the catalysts is known in the art and the known methods are applicable to the preparation of the catalyst of this invention. Methods of preparing the catalyst include impregnating the carrier with a silver compound and performing a reduction to form metallic silver particles. Reference may be made, for example, to US-A-5380697, US-A-5739075, US-B-6368998, US-2002/0010094 A1, EP-A-266015, WO-00/15333, WO-00/15334 and WO-00/15335.

The impregnation may include impregnation with a solution of which the pH has a value above 12, for example 13 or 13.2 or above. This may be accomplished by the addition of a base to the impregnation solution, for example lithium hydroxide, cesium hydroxide or a tetraalkylammonium hydroxide, such as tetramethylammonium hydroxide or tetraethylammonium hydroxide, in sufficient quantity. Dependent of the composition of the impregnation solution, a quantity of base in the range of from 20 to 70 mmole/kg catalyst, for example 30, 40, 50 or 60 mmole/kg catalyst may be sufficient to achieve a sufficiently high pH.

The reduction of cationic silver to metallic silver may be accomplished during a step in which the catalyst is dried, so that the reduction as such does require a separate process step. This may be the case if the impregnation solution comprises a reducing agent, for example, an oxalate, as described in the Examples hereinafter.

The catalyst preferably comprises silver, and a further element or compound thereof. Eligible further elements may be selected from the group of nitrogen, sulfur, phosphorus, boron, fluorine, Group IA metals, Group IIA metals, rhenium, molybdenum, tungsten, chromium, titanium, hafnium, zirconium, vanadium, thallium, thorium, tantalum, niobium, gallium and germanium and mixtures thereof. Preferably the Group IA metals are selected from lithium, potassium, rubidium and cesium. Most preferably the Group IA metal is lithium, potassium and/or cesium. Preferably the Group IIA metals are selected from calcium and barium. Where possible, the further element may suitably be provided as an oxyanion, for example, as a sulfate, borate, perrhenate, molybdate or nitrate, in salt or acid form.

It is preferred to employ the carrier of this invention in the preparation of a highly selective catalyst. The highly selective silver-based catalysts may comprise, in addition to silver, one or more of rhenium, molybdenum, tungsten, a Group IA metal, and a nitrate- or nitrite-forming compound, which may each be present in a quantity of from 0.01 to 500 mmole/kg, calculated as the element (rhenium, molybdenum, tungsten, the Group IA metal or nitrogen) on the total catalyst. The nitrate- or nitrite-forming compounds and particular selections of nitrate- or nitrite-forming compounds are as defined hereinafter. The nitrate- or nitrite-forming compound is in particular a Group IA metal nitrate or a Group IA metal nitrite. Rhenium, molybdenum, tungsten or the nitrate- or nitrite-forming compound may suitably be provided as an oxyanion, for example as a perrhenate, molybdate, tungstate or nitrate, in salt or acid form.

Of special preference are the highly selective catalysts which comprise rhenium in addition to silver. Such catalysts are known from EP-A-266015, US-A-4761394 and US-A-4766105. Broadly, they comprise silver, rhenium or compound thereof, the further element (as defined hereinbefore, in particular tungsten, molybdenum and/or a Group IA metal, in particular lithium and/or cesium) other than rhenium or compound thereof, and optionally a rhenium co-promoter. The rhenium co-promoter may be selected from one or more of sulfur, phosphorus, boron, and compounds thereof.

Preferred amounts of the components of the catalysts are, when calculated as the element, relative to the weight of the catalyst:
- silver from 10 to 500 g/kg,
- rhenium from 0.01 to 50 mmole/kg, if present,
- the further element or elements, if present, each from 0.1 to 500 mmole/kg, and,
- the rhenium co-promoter from 0.1 to 30 mmole/kg, if present.

With respect to silver, this metal is present preferably in an amount of 50 to 500 g/kg, more preferably 50 to 400 g/kg, in particular 50 to 250 g/kg, for example 105 g/kg, or 120 g/kg, or 145 g/kg, or 191 g/kg, or 200 g/kg. Rhenium may preferably be present in an amount of from 0.1 to 10 mmoles/kg, for example 2 mmoles/kg, or 3 mmoles/kg, or 5 mmoles/kg. The further element or elements may each be present in a preferred amount of from 0.5 to 100 mmole/kg. For example, tungsten may typically be present in an amount in the range of from 0.5 to 20 mmole/kg, such as 1 mmole/kg, or 1.5 mmoles/kg, or 5 mmole/kg, or 15 mmole/kg; molybdenum may typically be present in an amount in the range of from 1 to 40 mmole/kg, such as 2.3 mmole/kg, or 12 mmole/kg, or 25 mmole/kg; and the alkali metal may each typically be present in amount of from 5 to 100 mmole/kg. Suitable amounts for lithium are for example 5 mmole/kg, or 10 mmole/kg, or 22.2 mmole/kg, or 30 mmole/kg, or 40 mmole/kg, or 50 mmole/kg. Suitable amounts for cesium are for example 5 mmole/kg, or 5.3 mmole/kg, or 5.4 mmole/kg, or 7.2 mmole/kg, or 10.3 mmole/kg, or 10.5 mmole/kg, or 33 mmole/kg, or 47 mmole/kg.

Although the present epoxidation process may be carried out in many ways, it is preferred to carry it out as a gas phase process, i.e. a process in which the feed is contacted in the gas phase with the catalyst which is present as a solid material, typically in a packed bed. Generally the process is carried out as a continuous process.

The olefin for use in the present epoxidation process may be any olefin, such as an aromatic olefin, for example styrene, or a di-olefin, whether conjugated or not, for example 1,9-decadiene or 1,3-butadiene. Typically, the olefin is a monoolefin, for example 2-butene or isobutene. Preferably, the olefin is a mono-α-olefin, for example 1-butene or propylene. The most preferred olefin is ethylene.

The olefin concentration in the feed may be selected within a wide range. Typically, the olefin concentration in the feed will be at most 80 mole%, relative to the total feed. Preferably, it will be in the range of from 0.5 to 70 mole%, in particular from 1 to 60 mole%, on the same basis. As used herein, the feed is considered to be the composition which is contacted with the catalyst.

The present epoxidation process may be air-based or oxygen-based, see "Kirk-Othmer Encyclopedia of Chemical Technology", 3rd edition, Volume 9, 1980, pp. 445-447. In the air-based process air or air enriched with oxygen is employed as the source of the oxidizing agent while in the oxygen-based processes high-purity (at least 95 mole%) oxygen is employed as the source of the oxidizing agent. Presently most epoxidation plants are oxygen-based and this is a preferred embodiment of the present invention.

The oxygen concentration in the feed may be selected within a wide range. However, in practice, oxygen is generally applied at a concentration which avoids the flammable regime. Typically, the concentration of oxygen applied will be within the range of from 1 to 15 mole%, more typically from 2 to 12 mole% of the total feed.

In order to remain outside the flammable regime, the concentration of oxygen in the feed may be lowered as the concentration of the olefin is increased. The actual safe operating ranges depend, along with the feed composition, also on the reaction conditions such as the reaction temperature and the pressure.

A reaction modifier may be present in the feed for increasing the selectively, suppressing the undesirable oxidation of olefin or olefin oxide to carbon dioxide and water, relative to the desired formation of olefin oxide. Many organic compounds, especially organic halides and organic nitrogen compounds, may be employed as the reaction modifier. Nitrogen oxides, hydrazine, hydroxylamine or ammonia may be employed as well. It is frequently considered that under the operating conditions of olefin epoxidation the nitrogen containing reaction modifiers are precursors of nitrates or nitrites, i.e. they are so-called nitrate- or nitrite-forming compounds (cf. e.g. EP-A-0003642 and US-A-4822900).

Organic halides are the preferred reaction modifiers, in particular organic bromides, and more in particular organic chlorides. Preferred organic halides are chlorohydrocarbons or bromohydrocarbons. More preferably they are selected from the group of methyl chloride, ethyl chloride, ethylene dichloride, ethylene dibromide, vinyl chloride or a mixture thereof. Most preferred reaction modifiers are ethyl chloride and ethylene dichloride.

Suitable nitrogen oxides are of the general formula NOₓ wherein x is in the range of from 1 to 2, and include for example NO, N₂O₃ and N₂O₄. Suitable organic nitrogen compounds are nitro compounds, nitroso compounds, amines, nitrates and nitrites, for example nitromethane, 1-nitropropane or 2-nitropropane. In preferred embodiments, nitrate- or nitrite-forming compounds, e.g. nitrogen oxides and/or organic nitrogen compounds, are used together with an organic halide, in particular an organic chloride.

The reaction modifiers are generally effective when used in low concentration in the feed, for example up to 0.1 mole%, relative to the total feed, for example from 0.01×10⁻⁴ to 0.01 mole%. In particular when the olefin is ethylene, it is preferred that the reaction modifier is present in the feed at a concentration of from 0.1×10⁻⁴ to 50×10⁻⁴ mole%, in particular from 0.3×10⁻⁴ to 30×10⁻⁴ mole%, relative to the total feed.

In addition to the olefin, oxygen and the reaction modifier, the feed may contain one or more optional components, such as carbon dioxide, inert gases and saturated hydrocarbons. Carbon dioxide is a by-product in the epoxidation process. However, carbon dioxide generally has an adverse effect on the catalyst activity. Typically, a concentration of carbon dioxide in the feed in excess of 25 mole%, preferably in excess of 10 mole%, relative to the total feed, is avoided. A concentration of carbon dioxide as low as 1 mole% or lower, relative to the total feed, may be employed. Inert gases, for example nitrogen or argon, may be present in the feed in a concentration of from 30 to 90 mole%, typically from 40 to 80 mole%. Suitable saturated hydrocarbons are methane and ethane. If saturated hydrocarbons are present, they may be present in a quantity of up to 80 mole%, relative to the total feed, in particular up to 75 mole%. Frequently they are present in a quantity of at least 30 mole%, more frequently at least 40 mole%. Saturated hydrocarbons may be added to the feed in order to increase the oxygen flammability limit.

The epoxidation process may be carried out using reaction temperatures selected from a wide range. Preferably the reaction temperature is in the range of from 150 to 325 °C, more preferably in the range of from 180 to 300 °C.

The epoxidation process is preferably carried out at a reactor inlet pressure in the range of from 1000 to 3500 kPa. "GHSV" or Gas Hourly Space Velocity is the unit volume of gas at normal temperature and pressure (0 °C, 1 atm, i.e. 101.3 kPa) passing over one unit volume of packed catalyst per hour. Preferably, when the epoxidation process is as a gas phase process involving a packed catalyst bed, the GHSV is in the range of from 1500 to 10000 Nl/(l.h). Preferably, the process is carried out at a work rate in the range of from 0.5 to 10 kmole olefin oxide produced per m³ of catalyst per hour, in particular 0.7 to 8 kmole olefin oxide produced per m³ of catalyst per hour, for example 5 kmole olefin oxide produced per m³ of catalyst per hour. As used herein, the work rate is the amount of the olefin oxide produced per unit volume of catalyst per hour and the selectivity is the molar quantity of the olefin oxide formed relative to the molar quantity of the olefin converted.

The olefin oxide produced may be recovered from the reaction mixture by using methods known in the art, for example by absorbing the olefin oxide from a reactor outlet stream in water and optionally recovering the olefin oxide from the aqueous solution by distillation. At least a portion of the aqueous solution containing the olefin oxide may be applied in a subsequent process for converting the olefin oxide into a 1,2-diol or a 1,2-diol ether.

The olefin oxide produced in the epoxidation process may be converted into a 1,2-diol, a 1,2-diol ether, or an alkanolamine. As this invention leads to a more attractive process for the production of the olefin oxide, it concurrently leads to a more attractive process which comprises producing the olefin oxide in accordance with the invention and the subsequent use of the obtained olefin oxide in the manufacture of the 1,2-diol, 1,2-diol ether, and/or alkanolamine.

The conversion into the 1,2-diol or the 1,2-diol ether may comprise, for example, reacting the olefin oxide with water, suitably using an acidic or a basic catalyst. For example, for making predominantly the 1,2-diol and less 1,2-diol ether, the olefin oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-diol ethers in the reaction mixture is increased. The 1,2-diol ethers thus produced may be a di-ether, tri-ether, tetra-ether or a subsequent ether. Alternative 1,2-diol ethers may be prepared by converting the olefin oxide with an alcohol, in particular a primary alcohol, such as methanol or ethanol, by replacing at least a portion of the water by the alcohol.

The conversion into the alkanolamine may comprise, for example, reacting the olefin oxide with ammonia. Anhydrous or aqueous ammonia may be used, although anhydrous ammonia is typically used to favour the production of monoalkanolamine. For methods applicable in the conversion of the olefin oxide into the alkanolamine, reference may be made to, for example US-A-4845296.

The 1,2-diol and the 1,2-diol ether may be used in a large variety of industrial applications, for example in the fields of food, beverages, tobacco, cosmetics, thermoplastic polymers, curable resin systems, detergents, heat transfer systems, etc. The alkanolamine may be used, for example, in the treating ("sweetening") of natural gas.

Unless specified otherwise, the low-molecular weight organic compounds mentioned herein, for example the olefins, 1,2-diols, 1,2-diol ethers, alkanolamines and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

Having generally described the invention, a further understanding may be obtained by reference to the following examples, which are provided for purposes of illustration only and are not intended to be limiting unless otherwise specified.

### EXAMPLES

### Preparation of Carriers

A carrier (designated hereinafter as "Carrier A") was made by mixing the following ingredients:
1. 67.4 parts by weight (pbw) of an α-alumina with d₅₀ of 29 µm;
2. 29 pbw of an α-alumina with d₅₀ of 3 µm;
3. 3 pbw of aluminum oxide (in the form of boehmite);
4. 0.5 pbw of silica (in the form of ammonia stabilized silica sol); and
5. 0.1 pbw of sodium oxide (in the form of sodium acetate).

To this mixture were added 5 %w, relative to the mixture weight, of petroleum jelly and 9 %w, relative to the mixture weight, of burnout material and 0.1 %w, relative to the mixture weight, of boric acid. Water (about 30 %w, relative to the mixture weight) was then added in an amount to make the mixture extrudable and this mixture was then extruded to form formed bodies in the form of hollow cylinders that are about 8 mm in diameter and 8 mm long. These were then dried and fired in a kiln at 1425 °C, for 4 hours in air to produce Carrier A. As regards procedures followed in this carrier preparation, reference may be made to US-A-5100859.

A second carrier (hereinafter "Carrier B") was made by the same procedure as Carrier A, except that 14 %w of burnout material was used, instead of 9 %w.

A third carrier (hereinafter "Carrier C") was made by the same procedure as Carrier A, except that:
- 20 pbw of the α-alumina with d₅₀ of 3 µm was used instead of 29 pbw;
- 76.4 pbw of an α-alumina with d₅₀ of 16 µm was used, instead of the α-alumina with d₅₀ of 28 µm;
- 8 %w of burnout material was used, instead of 9 %w; and
- 14 %w, relative to the mixture weight, of the petroleum jelly was used, instead of 5 %w.

For comparative purposes, an α-alumina was prepared according to the process as described in Example 1 of US-A-5100859 (hereinafter "Carrier D").

The carriers exhibited characteristics as indicated in Table I. The pore size distribution is specified as the volume fraction (%v) and the volume (ml/g) of the pores having diameters in the specified ranges (< 0.2 µm, 0.2-10 µm, and >10 µm), relative to the total pore volume. "Pore volume" represents the total pore volume.

**TABLE I**

| Carrier | Surface area (m²/g) | Water absorption (g/g) | Pore volume (ml/g) | Pore size distribution | | |
|---|---|---|---|---|---|---|
| | | | | <0.2 µm (%v) | 0.2-10 µm (%v; ml/g) | >10 µm (%v) |
| A | 2.04 | 0.42 | 0.41 | 5 | 92; 0.37 | 3 |
| B *) | 2.11 | 0.49 | 0.42 | 9 | 72; 0.30 | 19 |
| C | 2.51 | 0.55 | 0.56 | 3 | 95; 0.53 | 2 |
| D *) | 0.73 | 0.40 | 0.40 | < 1 | 64; 0.26 | 36 |

| | | | | | | |
|---|---|---|---|---|---|---|
| *) comparative | | | | | | |

### Preparation of catalysts

A silver-amine-oxalate stock solution was prepared by the following procedure:
415 g of reagent-grade sodium hydroxide were dissolved in 2340 ml de-ionized water and the temperature was adjusted to 50 °C.
1699 g high purity "Spectropure" silver nitrate was dissolved in 2100 ml de-ionized water and the temperature was adjusted to 50 °C.

The sodium hydroxide solution was added slowly to the silver nitrate solution, with stirring, while maintaining a solution temperature of 50 °C. This mixture was stirred for 15 minutes, then the temperature was lowered to 40 °C.

Water was removed from the precipitate created in the mixing step and the conductivity of the water, which contained sodium and nitrate ions, was measured. An amount of fresh deionized water equal to the amount removed was added back to the silver solution. The solution was stirred for 15 minutes at 40 °C. The process was repeated until the conductivity of the water removed was less than 90 µmho/cm. 1500 ml fresh deionized water was then added.

630 g of high-purity oxalic acid dihydrate were added in approximately 100 g increments. The temperature was keep at 40 °C and the pH was kept above 7.8.

Water was removed from this mixture to leave a highly concentrated silver-containing slurry. The silver oxalate slurry was cooled to 30 °C.

699 g of 92 %w ethylenediamine (8% de-ionized water) was added while maintaining a temperature no greater than 30 °C. The resulting solution contained approximately 27-33 %w silver.

Enough 45 %w aqueous cesium hydroxide and water was added to this solution to give a finished catalyst having 14.5 %w silver and a cesium loading which provides the optimum selectivity. Actual cesium loadings have been specified in Table II, hereinafter.

Carriers A, B and D, prepared as indicated under the heading "Preparation of Carriers", were impregnated with the impregnation solution and dried, as follows, to form Catalyst A (according to the invention), Catalyst B (for comparison) and Catalyst D (for comparison), respectively.

A carrier sample of approximately 30 g was placed under a 25 mm Hg vacuum for 1 minute at ambient temperature. Approximately 50 g of the impregnating solution was then introduced to submerse the carrier, and the vacuum was maintained at 25 mm Hg for an additional 3 minutes. The vacuum was then released and the excess impregnating solution was removed from the catalyst pre-cursor by centrifugation at 500 rpm for two minutes. The catalyst pre-cursor was then dried while being shaken at 250 °C for 5.5 minutes in a stream of air.

The catalysts were used to produce ethylene oxide from ethylene and oxygen. To do this, 1.5 to 2 g of crushed catalyst were loaded into a stainless steel U-shaped tube. The tube was immersed in a molten metal bath (heat medium) and the ends were connected to a gas flow system. The weight of catalyst used and the inlet gas flow rate (0.28 Nl/minute) were adjusted to give a gas hourly space velocity of 6800 Nl/(l.h), as calculated for un-ground bulk catalyst. The inlet gas pressure was 1530 kPa.

The gas mixture passed through the catalyst bed, in a "once-through" operation, during the entire test run including the start-up, consisted of 25 %v ethylene, 7 %v oxygen, 5 %v carbon dioxide, 63 %v nitrogen and 2.0 to 6.0 ppmv ethyl chloride.

The initial reactor temperature was 180 °C and this was ramped up at a rate of 10 °C per hour to 225 °C and then adjusted so as to achieve a constant ethylene oxide content of 1.5 %v in the outlet gas stream at an ethyl chloride concentration of 2.5 ppmv. Performance data at this conversion level are usually obtained when the catalyst has been on stream for a total of at least 1-2 days.

The initial performance values for selectivity and temperature are reported in Table II, below. A lower temperature needed to accomplish a certain ethylene oxide content in the outlet gas stream is indicative for a higher activity of the catalyst. - 29 -

**TABLE II**

| Catalyst | Cesium content (mmoles/kg) | Selectivity (%) | Temperature ( °C) |
|---|---|---|---|
| A *) | 5.4 | 82.5 | 224 |
| B **) | 5.3 | 81.9 | 232 |
| D **) | 3.0 | 81.9 | 240 |

| | | | |
|---|---|---|---|
| *) invention **) comparative | | | |

Additional catalysts may be obtained by impregnating separate samples of carrier C, such that the additional catalysts contain, per kg of catalyst, for example, 191 g silver, 3 mmoles rhenium, 1.5 mmoles tungsten, 10.5 mmoles cesium and 40 mmoles lithium, or 200 g silver, 5 mmoles rhenium, 5 mmoles tungsten, 7.2 mmoles cesium and 22.2 mmoles lithium, or 145 g silver, 2 mmoles rhenium, 2.3 mmoles molybdenum, 10.3 mmoles cesium and 30 mmoles lithium. These catalysts may be used to produce ethylene oxide from ethylene and oxygen.

## Claims

1. A catalyst which comprises a carrier and silver deposited on the carrier in a quantity of at least 10 g/kg, relative to the weight of the catalyst, which carrier has a surface area in the range of from 1.6 m²/g to at most 2.9 m²/g, and a pore size distribution such that pores with diameters in the range of from 0.2 to 10 µm represent more than 80 % of the total pore volume and such pores together provide a pore volume of at least 0.27 ml/g, relative to the weight of the carrier, wherein the carrier comprises at least 95%w α-alumina .

2. A catalyst as claimed in claim 1 which comprises, deposited on the carrier in addition to silver, one or more further elements selected from the group of nitrogen, sulfur, phosphorus, boron, fluorine, Group IA metals, Group IIA metals, rhenium, molybdenum, tungsten, chromium, titanium, hafnium, zirconium, vanadium, thallium, thorium, tantalum, niobium, gallium and germanium and mixtures thereof.

3. A catalyst as claimed in claim 1 or 2 which comprises, deposited on the carrier in addition to silver, one or more of rhenium, molybdenum, tungsten, a Group IA metal, and a nitrate-or nitrite-forming compound.

4. A catalyst as claimed in claim 2 or 3 wherein the Group IA metals are selected from lithium, potassium, rubidium and cesium.

5. A catalyst as claimed in any one of claims 1-4
wherein the catalyst comprises rhenium in a quantity in the range of 0.01 to 500 mmole/kg, calculated as the element relative to the weight of the catalyst.

6. A catalyst as claimed in any one of claims 1-5
wherein the catalyst comprises silver, rhenium or compound thereof, a further element selected from tungsten, molybdenum and/or Group IA metal and a rhenium co-promoter selected from one or more of sulfur, phosphorus, boron, and compounds thereof.

7. A catalyst as claimed in claim 6, wherein the catalyst comprises, when calculated as the element, relative to the weight of the catalyst:
- silver from 10 to 500 g/kg;
- rhenium from 0.01 to 50 mmole/kg;
- the further element or elements, each from 0.1 to 500 mmole/kg; and
- the rhenium co-promoter from 0.1 to 30 mmole/kg.

8. A catalyst as claimed in any one of claims 1-7
wherein the carrier has a pore size distribution such that the pores with diameters in the range of from 0.2 to 10 µm represent more than 80 % of the total pore volume and such pores together provide a pore volume of at least 0.28 ml/g, relative to the weight of the carrier; the pores with diameters greater than 10 µm represent less than 15 % of the total pore volume; and the pores with diameters less than 0.2 µm represent less than 10 % of the total pore volume.

9. A catalyst as claimed in any one of claims 1-8 wherein the carrier has a pore size distribution such that the pores with diameters in the range of from 0.2 to 10 µm represent more than 90 % of the total pore volume and such pores together provide a pore volume of at least 0.3 ml/g, relative to the weight of the carrier; the pores with diameters greater than 10 µm represent less than 10 % of the total pore volume; and the pores with diameters less than 0.2 µm represent less than 7 % of the total pore volume.

10. A catalyst as claimed in any one of claims 1-9 wherein the carrier has a total pore volume in the range of from 0.3 to 0.7 ml/g.

11. A catalyst as claimed in any one of claims 1-10 wherein the carrier has a surface area in the range of from 1.4 m²/g to 2.6 m²/g.

12. A catalyst as claimed in any one of claims 1-11 wherein silver is deposited on the carrier in a quantity of from 50 to 500 g/kg, in particular 50 to 400 g/kg, more in particular 50 to 250 g/kg, relative to the weight of the catalyst.

13. A catalyst as claimed in any one of claims 1-12 wherein the carrier comprises a bond material which is based on a silica-containing composition comprising an inhibitor for the formation of crystalline silica-containing compositions that is an alkali metal compound.

14. A catalyst as claimed in claim 13 wherein the bond material is based on an alumina hydrate, an amorphous silica compound, and an alkali metal compound.

15. A process for the preparation of a catalyst as claimed in any one of claims 1-14, which process comprises:
selecting a carrier having a surface area in the range of from 1.6 m²/g to at most 2.9 m²/g, and a pore size distribution such that pores with diameters in the range of from 0.2 to 10 µm represent more than 80 % of the total pore volume and such pores together provide a pore volume of at least 0.27 ml/g, relative to the weight of the carrier, wherein the carrier comprises at least 95 %w α-alumina;
depositing at least silver on the carrier in a quantity of at least 10 g/kg, relative to the weight of the catalyst, and, if necessary, one or more further elements selected from the group of nitrogen, sulfur, phosphorus, boron, fluorine, Group IA metals, Group IIA metals, rhenium, molybdenum, tungsten, chromium, titanium, hafnium, zirconium, vanadium, thallium, thorium, tantalum, niobium, gallium and germanium and mixtures thereof.

16. A process as claimed in claim 15, wherein the carrier has been obtained by a method which comprises forming a mixture comprising:
a) from 50 to 90 % w of a first particulate α-alumina having an average particle size (d₅₀) of from more than 10 up to 100 µm; and
b) from 10 to 50 % w of a second particulate α-alumina having an average particle size (d₅₀) of from 1 to 10 µm;
%w being based on the total weight of α-alumina in the mixture; and firing the mixture to form the carrier wherein the average particle size represents a particle diameter at which there are equal spherical equivalent volumes of particles larger and particles smaller than the stated average particle size.

17. A process as claimed in claim 16, wherein
the mixture comprises:
a) from 65 to 75 %w, relative to the total weight of α-alumina in the mixture, of a first particulate α-alumina having an average particle size (d50) of from 11 to 60 µm;
b) from 25 to 35 %w, relative to the total weight of α-alumina in the mixture, of a second particulate α-alumina having an average particle size (d50) of from 2 to 6 µm;
c) from 2 to 5 %w of an alumina hydrate, calculated as aluminum oxide relative to the total weight of α-alumina in the mixture;
d) from 0.2 to 0.8 %w of an amorphous silica compound, calculated as silicon oxide relative to the total weight of α-alumina in the mixture; and
e) from 0.05 to 0.3 %w of an alkali metal compound, calculated as the alkali metal oxide relative to the total weight of α-alumina in the mixture,
and the mixture is shaped into formed bodies and the formed bodies are fired at a temperature of from 1250 to 1500°C.

18. A process for the epoxidation of an olefin, which process comprises reacting an olefin with oxygen in the presence of a catalyst as claimed in any one of claims 1-14.

19. A process as claimed in claim 18 wherein the olefin is ethylene.

20. A method of making a 1,2 diol, a 1,2-diol ether or an alkanolamine comprising:
(i) reacting an olefin with oxygen in the presence of a catalyst as claimed in any one of claims 1-14 to produce an olefin oxide; and
(ii) converting the olefin oxide into the 1,2-diol, the 1,2-diol ether or the alkanolamine.

## Patentansprüche

1. Katalysator, der einen Träger und auf dem Träger in einer Menge von mindestens 10 g/kg, bezogen auf das Gewicht des Katalysators, aufgebrachtes Silber umfasst, wobei der Träger eine Oberfläche in dem Bereich von 1,6 m²/g bis höchstens 2,9 m²/g aufweist und eine Porengrößenverteilung derart, dass die Poren mit Durchmessern in dem Bereich von 0,2 bis 10 µm mehr als 80 % des gesamten Porenvolumens darstellen und solche Poren zusammen ein Porenvolumen von mindestens 0,27 ml/g bereitstellen, bezogen auf das Gewicht des Trägers, wobei der Träger mindestens 95 Gew.-% α-Aluminiumoxid umfasst.

2. Katalysator wie beansprucht in Anspruch 1, der aufgebracht auf dem Träger zusätzlich zu dem Silber ein oder mehrere weitere Elemente auswählt aus der Gruppe aus Stickstoff, Schwefel, Phosphor, Bor, Fluor, Gruppe IA Metallen, Gruppe IIA Metallen, Rhenium, Molybdän, Wolfram, Chrom, Titan, Hafnium, Zirkonium, Vanadium, Thallium, Thorium, Tantal, Niob, Gallium und Germanium und Mischungen davon umfasst.

3. Katalysator wie beansprucht in Anspruch 1 oder 2, der aufgebracht auf dem Träger zusätzlich zu dem Silber eines oder mehrere aus Rhenium, Molybdän, Wolfram, einem Gruppe IA Metall, und einer nitrat-oder nitritbildenden Verbindung umfasst.

4. Katalysator wie beansprucht in Anspruch 2 oder 3, wobei die Gruppe IA Metalle ausgewählt sind aus Lithium, Kalium, Rubidium und Cäsium.

5. Katalysator wie beansprucht in einem der Ansprüche 1-4, wobei der Katalysator Rhenium in einer Menge in dem Bereich von 0,01 bis 500 mmol/kg, berechnet als das Element bezogen auf das Gewicht des Katalysators, umfasst.

6. Katalysator wie beansprucht wie in einem der Ansprüche 1-5, wobei der Katalysator Silber, Rhenium oder Verbindungen davon, ein weiteres Element ausgewählt aus Wolfram, Molybdän und/oder einem Gruppe IA Metall und einen Rhenium Co-Promoter, ausgewählt aus einem oder mehreren aus Schwefel, Phosphor, Bor und Verbindungen davon, umfasst.

7. Katalysator wie beansprucht in Anspruch 6, wobei der Katalysator, wenn berechnet als das Element, bezogen auf das Gewicht des Katalysators, umfasst:
- Silber von 10 bis 500 g/kg;
- Rhenium von 0,01 bis 50 mmol/kg;
- das weitere Element oder die weiteren Elemente, jedes von 0,1 bis 500 mmol/kg; und
- den Rhenium Co-Promoter von 0,1 bis 30 mmol/kg.

8. Katalysator wie beansprucht in einem der Ansprüche 1-7, wobei der Träger eine Porengrößenverteilung derart aufweist, dass die Poren mit Durchmessern in dem Bereich von 0,2 bis 10 µm mehr als 80 % des gesamten Porenvolumens darstellen und solche Poren zusammen ein Porenvolumen von mindestens 0,28 ml/g, bezogen auf das Gewicht des Trägers, bereitstellen; die Poren mit Durchmessern von größer als 10 µm weniger als 15 % des gesamten Porenvolumens darstellen; und die Poren mit Durchmessern von weniger als 0,2 µm weniger als 10 % des gesamten Porenvolumens darstellen.

9. Katalysator wie beansprucht in einem der Ansprüche 1-8, wobei der Träger eine Porengrößenverteilung derart aufweist, dass die Poren mit Durchmessern in dem Bereich von 0,2 bis 10 µm mehr als 90 % des gesamten Porenvolumens darstellen und solche Poren zusammen ein Porenvolumen von mindestens 0,3 ml/g, bezogen auf das Gewicht des Trägers, bereitstellen; die Poren mit Durchmessern von größer als 10 µm weniger als 10 % des gesamten Porenvolumens darstellen; und die Poren mit Durchmessern von weniger als 0,2 µm weniger als 7 % des gesamten Porenvolumens darstellen.

10. Katalysator wie beansprucht in einem der Ansprüche 1-9, wobei der Träger ein gesamtes Porenvolumen in dem Bereich von 0,3 bis 0,7 ml/g aufweist.

11. Katalysator wie beansprucht in einem der Ansprüche 1-10, wobei der Träger eine Oberfläche in dem Bereich von 1,4 m²/g bis 2,6 m²/g aufweist.

12. Katalysator wie beansprucht in einem der Ansprüche 1-11, wobei Silber in einer Menge von 50 bis 500 g/kg, insbesondere 50 bis 400 g/kg, ganz besonders 50 bis 250 g/kg, bezogen auf das Gewicht des Katalysators, auf dem Träger aufgebracht ist.

13. Katalysator wie beansprucht in einem der Ansprüche 1-12, wobei der Träger ein Bindungsmaterial umfasst, das auf einer Silica-enthaltenden Zusammensetzung basiert, umfassend einen Inhibitor für die Bildung von kristallinen Silica-enthaltenen Verbindungen, der eine Alkalimetall-Verbindung ist.

14. Katalysator wie beansprucht in Anspruch 13, wobei das Bindungsmaterial auf einem Aluminiumoxidhydrat, einer amorphen Silica-Verbindung, und einer Alkalimetall-Verbindung basiert ist.

15. Verfahren für die Herstellung eines Katalysators wie beansprucht in einem der Ansprüche 1-14, wobei das Verfahren umfasst:
Auswählen eines Trägers mit einer Oberfläche in dem Bereich von 1,6 m²/g bis höchstens 2,9 m²/g, und einer Porengrößenverteilung derart, dass die Poren mit Durchmessern in dem Bereich von 0,2 bis 10 µm mehr als 80 % des gesamten Porenvolumens darstellen und solche Poren zusammen ein Porenvolumen von mindestens 0,27 ml/g bereitstellen, bezogen auf das Gewicht des Trägers, wobei der Träger mindestens 95 Gew.-% α-Aluminiumoxid umfasst;
Aufbringen von mindestens Silber auf dem Träger in einer Menge von mindestens 10 g/kg, bezogen auf das Gewicht des Katalysators, und, wenn nötig, ein oder mehrere weitere Elemente ausgewählt aus der Gruppe aus Stickstoff, Schwefel, Phosphor, Bor, Fluor, Gruppe IA-Metallen, Gruppe IIA-Metallen, Rhenium, Molybdän, Wolfram, Chrom, Titan, Hafnium, Zirkonium, Vanadium, Thallium, Thorium, Tantal, Niob, Gallium und Germanium und
Mischungen davon.

16. Verfahren wie beansprucht in Anspruch 15, wobei der Träger durch eine Methode erhalten worden ist, die das Bilden einer Mischung umfassend:
a) von 50 bis 90 Gew.-% eines ersten teilchenförmigen α-Aluminiumoxids mit einer durchschnittlichen Partikelgröße (d₅₀) von mehr als 10 bis zu 100 µm; und
b) von 10 bis 50 Gew.-% eines zweiten teilchenförmigen α-Aluminiumoxids mit einer durchschnittlichen Partikelgröße (d₅₀) von 1 bis 10 µm; wobei Gew.-% auf dem gesamten Gewicht des α-Aluminiumoxids in der Mischung basiert ist; und Brennen der Mischung um den Träger zu bilden, wobei die durchschnittliche Partikelgröße einen Partikeldurchmesser darstellt, bei dem es gleich viele sphärische äquivalente Volumina von Partikeln, die größer sind, und Partikeln, die kleiner sind als der genannte durchschnittliche Partikeldurchmesser gibt,
umfasst.

17. Verfahren wie beansprucht in Anspruch 16, wobei die Mischung umfasst:
a) von 65 bis 75 Gew.-%, bezogen auf das gesamte Gewicht des α-Aluminiumoxids in der Mischung, eines ersten teilchenförmigen α-Aluminiumoxids mit einer durchschnittlichen Partikelgröße (d50) von 11 bis 60 µm;
b) von 25 bis 35 Gew.-%, bezogen auf das gesamte Gewicht des α-Aluminiumoxids in der Mischung, eines zweiten teilchenförmigen α-Aluminiumoxids mit einer durchschnittlichen Partikelgröße (d50) von 2 bis 6 µm;
c) von 2 bis 5 Gew.-% eines Aluminiumoxidhydrats, berechnet als Aluminiumoxid bezogen auf das gesamte Gewicht des α-Aluminiumoxids in der Mischung;
d) von 0,2 bis 0,8 Gew.-% einer amorphen Silica-Verbindung, berechnet als Siliziumoxid bezogen auf das gesamte Gewicht des α-Aluminiumoxids in der Mischung; und
e) von 0,05 bis 0,3 Gew.-% einer Alkalimetall-Verbindung, berechnet als das Alkalimetalloxid bezogen auf das gesamte Gewicht des α-Aluminiumoxids in der Mischung,
und die Mischung wird in Formkörper ausgeformt, und die Formkörper werden bei einer Temperatur von 1250 bis 1500°C gebrannt.

18. Verfahren für die Epoxidierung eines Olefins, wobei das Verfahren umfasst Reagierenlassen eines Olefins mit Sauerstoff in der Gegenwart eines Katalysators wie beansprucht in einem der Ansprüche 1-14.

19. Verfahren wie beansprucht in Anspruch 18, wobei das Olefin Ethylen ist.

20. Verfahren zum Herstellen eines 1,2-Diols, eines 1,2-Diol Ethers oder eines Alkanolamins umfassend:
(i) Reagierenlassen eines Olefins mit Sauerstoff in der Gegenwart eines Katalysators wie beansprucht in einem der Ansprüche 1-14, um ein Olefinoxid herzustellen; und
(ii)Umsetzen des Olefinoxids in das 1,2-Diol, in den 1,2-Diol Ether oder in das Alkanolamin.

## Revendications

1. Catalyseur qui comprend un support et de l'argent déposé sur le support dans une quantité d'au moins 10 g/kg, par rapport au poids du catalyseur, lequel support a un grammage dans la plage de 1,6 m²/g à au plus 2,9 m²/g, et une distribution de taille de pore telle que des pores avec des diamètres dans la plage de 0,2 à 10 µm représentent plus de 80 % du volume de pore total et ces pores confèrent ensemble un volume de pore d'au moins 0,27 mL/g, par rapport au poids du support, dans lequel le support comprend au moins 95 % en poids d'α-alumine.

2. Catalyseur selon la revendication 1, qui comprend, déposés sur le support en plus de l'argent, un ou plusieurs éléments supplémentaires choisis dans le groupe de l'azote, du soufre, du phosphore, du bore, du fluor, des métaux du groupe IA, des métaux du groupe IIA, du rhénium, du molybdène, du tungstène, du chrome, du titane, de l'hafnium, du zirconium, du vanadium, du thallium, du thorium, du tantale, du niobium, du gallium et du germanium et de leurs mélanges.

3. Catalyseur selon la revendication 1 ou 2, qui comprend, déposés sur le support en plus de l'argent, un ou plusieurs éléments parmi le rhénium, le molybdène, le tungstène, un métal du groupe IA, et un composé formant du nitrate ou du nitrite.

4. Catalyseur selon la revendication 2 ou 3, dans lequel les métaux du groupe IA sont choisis parmi le lithium, le potassium, le rubidium et le césium.

5. Catalyseur selon l'une quelconque des revendications 1 à 4, dans lequel le catalyseur comprend du rhénium dans une quantité dans la plage de 0,01 à 500 mmole/kg, calculé comme l'élément par rapport au poids du catalyseur.

6. Catalyseur selon l'une quelconque des revendications 1 à 5, dans lequel le catalyseur comprend de l'argent, du rhénium ou un de leurs composés, un élément supplémentaire choisi parmi le tungstène, le molybdène et/ou un métal du groupe IA et un copromoteur de rhénium choisi parmi un ou plusieurs éléments parmi le soufre, le phosphore, le bore, et leurs composés.

7. Catalyseur selon la revendication 6, dans lequel le catalyseur comprend, lorsqu'il est calculé comme l'élément, par rapport au poids du catalyseur :
- de l'argent de 10 à 500 g/kg ;
- du rhénium de 0,01 à 50 mmole/kg ;
- l'élément ou les éléments supplémentaires, chacun de 0,1 à 500 mmole/kg ; et
- le copromoteur de rhénium de 0,1 à 30 mmole/kg.

8. Catalyseur selon l'une quelconque des revendications 1 à 7, dans lequel le support a une distribution de taille de pore telle que les pores avec des diamètres dans la plage de 0,2 à 10 µm représentent plus de 80 % du volume de pore total et ces pores confèrent ensemble un volume de pore d'au moins 0,28 mL/g, par rapport au poids du support ; les pores avec des diamètres supérieurs à 10 µm représentent moins de 15 % du volume de pore total ; et les pores avec des diamètres inférieurs à 0,2 µm représentent moins de 10 % du volume de pore total.

9. Catalyseur selon l'une quelconque des revendications 1 à 8, dans lequel le support a une distribution de taille de pore telle que les pores avec des diamètres dans la plage de 0,2 à 10 µm représentent plus de 90 % du volume de pore total et ces pores confèrent ensemble un volume de pore d'au moins 0,3 mL/g, par rapport au poids du support ; les pores avec des diamètres supérieurs à 10 µm représentent moins de 10 % du volume de pore total ; et les pores avec des diamètres inférieurs à 0,2 µm représentent moins de 7 % du volume de pore total.

10. Catalyseur selon l'une quelconque des revendications 1 à 9, dans lequel le support a un volume de pore total dans la plage de 0,3 à 0,7 mL/g.

11. Catalyseur selon l'une quelconque des revendications 1 à 10, dans lequel le support a un grammage dans la plage de 1,4 m²/g à 2,6 m²/g.

12. Catalyseur selon l'une quelconque des revendications 1 à 11, dans lequel l'argent est déposé sur le support dans une quantité de 50 à 500 g/kg, en particulier 50 à 400 g/kg, plus particulièrement 50 à 250 g/kg, par rapport au poids du catalyseur.

13. Catalyseur selon l'une quelconque des revendications 1 à 12, dans lequel le support comprend un matériau de liaison qui est à base d'une composition contenant de la silice comprenant un inhibiteur pour la formation de compositions contenant de la silice cristalline qui est un composé de métal alcalin.

14. Catalyseur selon la revendication 13, dans lequel le matériau de liaison est à base d'un hydrate d'alumine, d'un composé de silice amorphe, et d'un composé de métal alcalin.

15. Procédé pour la préparation d'un catalyseur tel que revendiqué dans l'une quelconque des revendications 1 à 14, lequel procédé comprend :
le choix d'un support ayant un grammage dans la plage de 1,6 m²/g à au plus 2,9 m²/g, et une distribution de taille de pore telle que des pores avec des diamètres dans la plage de 0,2 à 10 µm représentent plus de 80 % du volume de pore total et ces pores confèrent ensemble un volume de pore d'au moins 0,27 mL/g, par rapport au poids du support, dans lequel le support comprend au moins 95 % en poids d'α-alumine ;
le dépôt au moins d'argent sur le support dans une quantité d'au moins 10 g/kg, par rapport au poids du catalyseur, et si nécessaire, d'un ou de plusieurs éléments supplémentaires choisis dans le groupe de l'azote, du soufre, du phosphore, du bore, du fluor, des métaux du groupe IA, des métaux du groupe IIA, du rhénium, du molybdène, du tungstène, du chrome, du titane, de l'hafnium, du zirconium, du vanadium, du thallium, du thorium, du tantale, du niobium, du gallium et du germanium et de leurs mélanges.

16. Procédé selon la revendication 15, dans lequel le support a été obtenu par un procédé qui comprend la formation d'un mélange comprenant :
a) de 50 à 90 % en poids d'une première α-alumine particulaire ayant une taille moyenne de particule (d₅₀) de plus de 10 jusqu'à 100 µm ; et
b) de 10 à 50 % en poids d'une seconde α-alumine particulaire ayant une taille moyenne de particule (d₅₀) de 1 à 10 µm ;
le % en poids étant basé sur le poids total d'α-alumine dans le mélange ; et la mise à feu du mélange afin de former le support, dans lequel la taille moyenne de particule représente un diamètre de particule auquel il y a des volumes équivalents sphériques égaux de particules plus grandes et de particules plus petites que la taille moyenne de particule énoncée.

17. Procédé selon la revendication 16, dans lequel le mélange comprend :
a) de 65 à 75 % en poids, par rapport au poids total d'α-alumine dans le mélange, d'une première α-alumine particulaire ayant une taille moyenne de particule (d₅₀) de 11 à 60 µm ;
b) de 25 à 35 % en poids, par rapport au poids total d'α-alumine dans le mélange, d'une seconde α-alumine particulaire ayant une taille moyenne de particule (d₅₀) de 2 à 6 µm ;
c) de 2 à 5 % en poids d'un hydrate d'alumine, calculé en tant qu'oxyde d'aluminium par rapport au poids total d'α-alumine dans le mélange ;
d) de 0,2 à 0,8 % en poids d'un composé de silice amorphe, calculé en tant qu'oxyde de silicium par rapport au poids total d'α-alumine dans le mélange ; et
e) de 0,05 à 0,3 % en poids d'un composé de métal alcalin, calculé en tant qu'oxyde de métal alcalin par rapport au poids total d'α-alumine dans le mélange,
et le mélange est formé en corps formés et les corps formés sont mis à feu à une température de 1 250 à 1 500 °C.

18. Procédé pour l'époxydation d'une oléfine, lequel procédé comprend la réaction d'une oléfine avec de l'oxygène en présence d'un catalyseur tel que revendiqué dans l'une quelconque des revendications 1 à 14.

19. Procédé selon la revendication 18, dans lequel l'oléfine est l'éthylène.

20. Procédé de fabrication d'un 1,2-diol, d'un 1,2-dioléther ou d'une alcanolamine comprenant :
(i) la réaction d'une oléfine avec de l'oxygène en présence d'un catalyseur tel que revendiqué dans l'une quelconque des revendications 1 à 14 afin de produire un oxyde d'oléfine ; et
(ii) la conversion de l'oxyde d'oléfine en le 1,2-diol, le 1,2-dioléther ou l'alcanolamine.
